# EUROPEAN PATENT APPLICATION

(11) **EP 3 892 188 A1**
(43) Date of publication of application: **13.10.2021**
(21) Application number: 19891915.1
(22) Date of filing: 04.12.2019
(51) Int. Cl.: A61B 5/02, A61B 5/0295, A61B 5/1455

(54) **BIOLOGICAL INFORMATION MEASUREMENT DEVICE**

(30) Priority: 04.12.2018 JP 2018227578
(71) Applicant: Asahi Kasei Kabushiki Kaisha, Tokyo 1000006 (JP)
(72) Inventor: YAMADA Masashi, Tokyo 100-0006 (JP); TSUBOI Yuka, Tokyo 100-0006 (JP)
(74) Representative: Hoffmann Eitle
(86) International application number: PCT/JP2019/047466
(87) International publication number: WO 2020/116527

(57) **Abstract**

A sensor of a biological information measurement device is easily and reliably attached to a living body with an appropriate pressing force. The biological information measurement device comprises a base, an adhering unit that is provided in a bottom surface portion of the base and sticks to a living body surface of a subject with a predetermined sticking force, a first sensor unit that is provided in a displaceable manner with respect to the base for measuring biological information of the subject, and a pressing unit for elastically pressing the first sensor unit to the living body surface against the predetermined sticking force in a case where the adhering unit sticks to the living body surface.

## Description

### Technical Field

The present invention relates to a biological information measurement device.

### Background Art

As one of the methods for non-invasively monitoring the biological information of a subject, there is Photoplethysmography (hereinafter, referred to as "PPG"). PPG is a method of monitoring changes in blood flow by irradiating the living body surface of a subject with light having a predetermined wavelength and measuring the time-series change in the light quantity transmitted through the living body surface. Since the blood flow is affected by a plurality of biological systems, by adopting PPG, it is possible to measure various biological indexes, such as pulse rate (HB), heart rate variability (HRV), vascular elasticity (RI), arterial oxygen saturation (SpO2), and local tissue oxygen saturation (rSO2). There are examples where a probe for PPG measurement is attached to a finger, an earlobe, or an arm (wrist).

For example, Patent Document 1 below discloses a device for measuring physiological parameters, such as PPG parameters of a subject, that comprises a fixture for fixing to the arm of the subject, a sensor for measuring the physiological parameters of the subject, a processor for converting measured values based on signals received from the sensor to form medical information, and a communication device for receiving the medical information from the processor and for transmitting the medical information.

Meanwhile, Patent Document 2 below discloses a biopotential measuring electrode that comprises a sticking sheet having a sticking surface fixed to a living body and having a through-hole formed at a substantially central portion, an electrode unit that is brought into close contact with the living body by pressing an elastic body through the through-hole of the sticking sheet, a holding unit that holds the electrode unit, and an engaging unit that attachably and detachably engages the holding unit with the sticking sheet.

Patent Document 3 below discloses a system including a processor that measures waveforms associated with arterial oxygen concentration and corrects artifacts associated with the movement of tissue site, by using a first physiological parameter associated with arterial oxygen concentration measured by a first sensor and a second physiological parameter associated with respiration measured by a second sensor.

### Citation List

### Patent Literature

Patent Document 1: JP 2005-511223 A
Patent Document 2: JP 3-228737 A
Patent Document 3: U.S. Pre-Grant Publication No. 2018/0235526

### Summary of Invention

### Technical Problem

In the device disclosed in Patent Document 1, since the sensor for measuring PPG is attached to the finger, earlobe, or arm of the subject, the sensor itself is required to be miniaturized, and is inconvenient to detect a weak biological signal. In the above-described device of the related art, while the PPG sensor is attached to the arm or the like of the subject, the electrode sensor is configured to be attached to the skin surface (hereinafter, referred to as "living body surface") of a living body for the measurement of the electrocardiogram, and thus, it was complicated to attach various sensors.

The PPG sensor needs to be attached to the skin while being pressed with a certain force in order to improve the measurement accuracy, and a band or a belt has been used to attach the PPG sensor to the arm or the like. Therefore, there has been no proposal to attach the PPG sensor to the living body surface, and even if there is a PPG sensor to be attached to the living body surface, there is a problem that it is complicated to attach the PPG sensor by using a band or the like.

The technology disclosed in Patent Document 2 is a device that presses an electrode arranged substantially at the center of the sticking sheet against a living body, and does not effectively measure PPG in the first place.

In the technology disclosed in Patent Document 3, although the first sensor is pressed against the tissue site to measure the arterial oxygen concentration, the first sensor is not efficiently pressed in relation to a sticking force of a sticking member.

An object of the present invention is to provide a mechanism capable of easily attaching a sensor on a living body surface with an appropriate pressing force and preventing the sensor from falling off.

Specifically, one object of the present invention is to provide a biological information measurement device having a mechanism capable of easily and reliably attaching a PPG sensor to a living body surface with an appropriate pressing force.

Another object of the present invention is to provide a biological information measurement device capable of sufficiently ensuring a contact area of a PPG sensor with a living body surface.

Another object of the present invention is to provide a biological information measurement device having a mechanism capable of effectively using a sticking force of a sticking member on a living body surface as a pressing force of a PPG sensor.

### Solution to Problem

The present invention for solving the above-described problems is configured to include the following specific matters or technical features of the invention.

Specifically, the present invention according to an aspect is a biological information measurement device comprises a base, a first sensor unit provided in a displaceable manner with respect to the base, and including a plurality of sensors that measures biological information of a subject; a pressing unit configured to elastically press the first sensor unit to a living body surface of the subject; and a plurality of adhering units, provided on the base, that adheres to the living body surface. The pressing unit may be configured to press the plurality of sensors of the first sensor unit against the base adhering to the living body surface by an adhesive force of the plurality of adhering units. Thus, the plurality of sensors can be easily attached to the living body surface with an appropriate pressing force. Since the tissue under the living body surface (skin) of the subject differs depending on the place, it is difficult to collect the biological information, but by providing the plurality of sensors, the biological information can be collected more reliably and the measurement accuracy can be improved.

The pressing unit may include a knocking unit configured to displace in response to a pressing operation, and a first elastic body configured to elastically bias the knocking unit. The first sensor unit is provided to be interlocked with the movement of the knocking unit. In response to a first pressing operation with respect to the knocking unit, the knocking unit advances and the first sensor unit at a retracted position advances and is positioned to protrude from a bottom surface portion of the base, and in response to a second pressing operation with respect to the knocking unit, the knocking unit retracts and the first sensor unit protruding from the bottom surface portion of the base moves to the retracted position.

More specifically, the pressing unit may comprise a cam main body having a cam groove and a tooth receiving unit, a knocking unit having a cam tooth slidably provided along the cam groove, and a first elastic body that elastically biases the knocking unit. The first sensor unit is provided to be interlocked with the movement of the knocking unit. In response to the first pressing operation with respect to the knocking unit, the cam tooth advances and is engaged with the tooth receiving unit, and accordingly, the first sensor unit at the retracted position advances and protrudes from the bottom surface portion of the base. In response to the second pressing operation with respect to the knocking unit, the cam tooth is disengaged from the tooth receiving unit and retracts along the cam tooth, and accordingly, the first sensor unit protruding from the bottom surface portion of the base moves to the retracted position.

The pressing unit may further include a second elastic body that elastically biases the first sensor unit.

The pressing unit may further include an adjusting unit that adjusts an amount of protrusion of the sensor unit with respect to a bottom surface of the base.

The first sensor unit may include one or a plurality of sensors for optically measuring PPG. Each of the plurality of sensors may be configured with a combination of one or a plurality of light emitting elements and one or a plurality of light receiving elements.

The adhering unit may include a second sensor unit, or the entire adhering unit may be the second sensor unit. Alternatively, the second sensor unit may be formed of a separate body from the adhering unit. The second sensor unit may include an electrode pad that measures a bioelectric signal.

The pressing unit may include a plurality of elastic bodies that elastically bias the first sensor unit. Each of the plurality of elastic bodies may be provided on a second surface opposite to a first surface of the first sensor unit on which the plurality of sensors is provided.

The base may be provided with a plurality of support units that support the plurality of adhering units. The plurality of support units may be configured with a flexible member.

A gravity center position with respect to the plurality of adhering units may be included in the first sensor unit in a virtual plane including the living body surface. Alternatively, the gravity center position with respect to the plurality of adhering units may substantially match a gravity center position with respect to the plurality of sensors. A gravity center position of the first sensor unit may substantially match a gravity center position of the plurality of elastic bodies.

The first sensor unit may include a board on which the plurality of sensors is mounted. The board may be a rigid board in whole or a part thereof, or may be a flexible board. The board may be formed in a curved portion, and the curved portion may be configured to come into contact with the living body surface following the shape of the living body surface.

### Advantageous Effects of Invention

According to the present invention, the sensor can be easily attached to the living body surface with an appropriate pressing force, and the biological information can be reliably measured without the sensor easily falling off.

According to the present invention, since the sensor can be easily attached to the living body surface with an appropriate pressing force, the contact area with the living body surface can be sufficiently ensured, unintended scattering of reflected light on the living body surface can be prevented, and/or the biological information can be more reliably measured as the biological signal is emphasized with an appropriate compression of arterial blood.

According to the present invention, the adhering unit having a relatively small adhering area effectively adheres to the undulations or irregularities of the living body surface, and accordingly, the attachment of the biological information measurement device can be reliably performed.

Furthermore, according to the present invention, since the gravity center of the sticking surface of the adhering unit is positioned within the board (first sensor unit) on which the sensor is mounted, the board can reliably press the living body surface with the pressing force of the elastic body against the sticking force of the adhering unit, and the biological information can be more reliably measured while the sensor is in contact with the living body surface with an appropriate pressing force.

Other technical features, objects, and effects or advantages of the present invention will be apparent by the following embodiments described with reference to the attached drawings.

### Brief Description of Drawings

FIG. 1 is an external upward perspective view showing an example of a biological information measurement device according to an embodiment of the present invention.
FIG. 2 is a bottom view showing an example of the biological information measurement device according to an embodiment of the present invention.
FIG. 3 is a view for illustrating a retractable pressing mechanism of the biological information measurement device according to an embodiment of the present invention, and is a view showing a state where a first sensor unit is at a retracted position.
FIG. 4 is a view for illustrating the retractable pressing mechanism of the biological information measurement device according to an embodiment of the present invention, and is a view showing a state where the first sensor unit is at a protruding position.
FIG. 5 is an external upward perspective view showing an example of the biological information measurement device according to an embodiment of the present invention.
FIG. 6 is an external downward perspective view showing an example of the biological information measurement device according to an embodiment of the present invention.
FIG. 7 is a side sectional view showing an example of a configuration of the biological information measurement device according to an embodiment of the present invention.
FIG. 8 is a view for illustrating an example of the biological information measurement device according to an embodiment of the present invention;
FIG. 9 is a plan view showing an example of a configuration of the biological information measurement device according to an embodiment of the present invention.
FIG. 10 is a side view of a base part of the biological information measurement device shown in FIG. 9.
FIG. 11 is a downward perspective view showing an example of an external configuration of the biological information measurement device according to an embodiment of the present invention.
FIG. 12 is a side view showing an example of an external configuration of the biological information measurement device illustrated in FIG. 11.
FIGS.13A to 13C are views for illustrating an example of a configuration of a bottom surface portion of the biological information measurement device according to the embodiment of the present invention;
FIG. 14 is a view for describing an example of a configuration of the bottom surface portion of the biological information measurement device according to an embodiment of the present invention.
FIG. 15 is a side view illustrating an example of an external configuration of the biological information measurement device according to an embodiment of the present invention.
FIG. 16 is a bottom view of the biological information measurement device shown in FIG. 15.
FIG. 17 shows views for illustrating examples of a positional relationship between the first sensor unit and an adhering unit of the biological information measurement device according to an embodiment of the present invention.
FIG. 18 is a view for illustrating an example of the positional relationship between the first sensor unit and the adhering unit of the biological information measurement device according to an embodiment of the present invention.
FIG. 19 shows views for illustrating examples of the positional relationship between the first sensor unit and the adhering unit in the biological information measurement device according to an embodiment of the present invention.
FIG. 20 shows views for illustrating examples of the positional relationship between the first sensor unit and the adhering unit in the biological information measurement device according to an embodiment of the present invention.

### Description of Embodiments

Hereinafter, embodiments of the present invention will be described with reference to the drawings. However, the embodiments described below are merely examples, and there is no intention of excluding the application of various modifications or technologies which are not specified below. The present invention can be implemented with various modifications (for example, combining each embodiment) without departing from the spirit of the present invention. In the description of the following drawings, the same or similar parts will be given the same or similar reference numerals. The drawings are schematic and do not necessarily match the actual dimensions or ratios. Even between drawings, there is a case where there are the parts having different dimensional relationships or ratios from each other.

### [First Embodiment]

In the present embodiment, an example of a biological information measurement device is described having a configuration in which a plurality of adhering units that adheres to a living body surface of a subject is provided, and a pressing mechanism or a pressing unit presses one or a plurality of PPG sensors against a base that adheres to the living body surface by an adhesive force of the plurality of adhering units. As an example of the pressing mechanism, a knocking mechanism is shown.

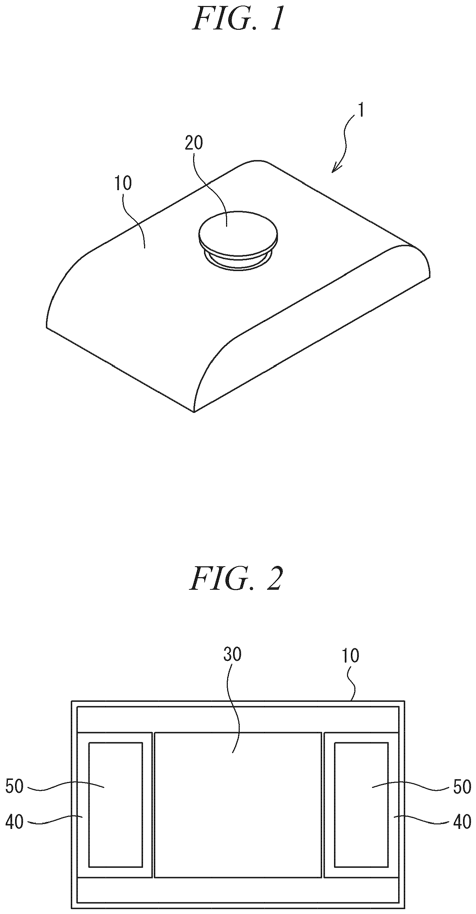

FIG. 1 is an external upward perspective view showing an example of the biological information measurement device according to an embodiment of the present invention. As shown in this figure, a schematic external view of the biological information measurement device 1 is formed by, for example, a flat housing or a base 10 in which at least one set of facing ridge portions on the top end portion side is rounded (a ridge R is formed) . In other words, the biological information measurement device 1 of the present example has a substantially rectangular shape in a plan view and a substantially semi-cylindrical shape (inverted U shape) in which the top end portion is flat in a front view. It should be noted that the shape of the base 10 is not limited to the present example, and may be a simple flat rectangular parallelepiped shape, a flat triangular shape, a disk shape, an elliptical disk shape, or the like. The base 10 may accommodate, for example, a control circuit, a communication module circuit, and the like therein.

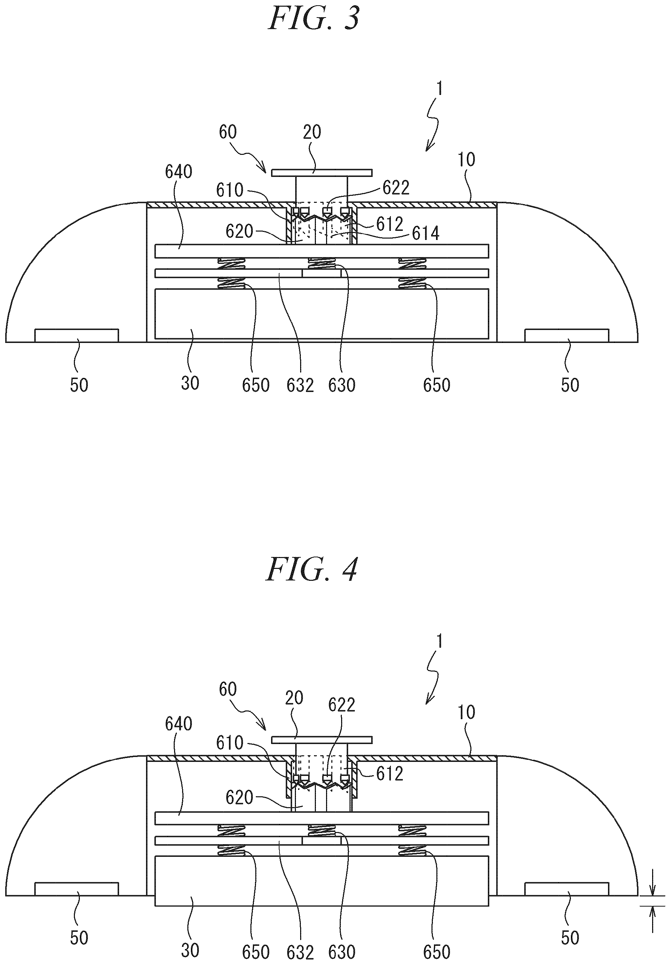

A knocking unit 20 having a head portion for a user (medical worker, subject, assistant, and the like) to press with, for example, his/her finger pulp is formed at a substantially central portion of the top end portion of the biological information measurement device 1. The biological information measurement device 1 is formed in a size that can be attached to the living body surface of the subject, and as an example, the base 10 has a width of approximately 80 mm, a depth of approximately 50 mm, and a height of approximately 18 mm, and the protrusion amount of the knocking unit 20 from the top end portion is approximately 5 mm. Examples of the living body surface part of the subject include the surface of the forehead, back, chest, abdomen, thighs, arms, neck, and hands. As will be described later, when the user presses down (presses) the knocking unit 20, the first sensor unit 30 provided on the bottom surface portion of the base 10 is configured to slightly protrude from the bottom surface portion (refer to FIG. 4).

FIG. 2 is a bottom view showing an example of the biological information measurement device according to the embodiment of the present invention. As illustrated in the drawing, on the bottom surface portion of the biological information measurement device 1, a first sensor unit 30 configured to be elastically displaceable with respect to the base 10 and an adhering unit 40 provided on the base 10 are provided. In the present disclosure, the term "displaceable" is used to include at least the fact that an object can move between two positions. The adhering unit 40 may be configured to include a second sensor unit 50, but in one example, the entire adhering unit 40 may be the second sensor unit 50. In another example, the adhering unit 40 and the second sensor unit 50 may be formed to be separated from each other. The first sensor unit 30 is arranged, for example, substantially at the center of the bottom surface portion of the base 10, and the adhering units 40 (or the second sensor units 50) are arranged on both sides of the first sensor unit 30 to sandwich the first sensor unit 30. Alternatively, the adhering units 40 (or the second sensor units 50) may be arranged, symmetrically or asymmetrically, and/or continuously or discretely, to surround the circumference of the first sensor unit 30.

The first sensor unit 30 is configured to include, for example, a board, and one or a plurality of sensors for measuring the biological information provided on the board. The board may be, for example, a rigid board in whole or a part thereof, or a flexible board. The one or the plurality of sensors are configured to include, for example, a PPG sensor for measuring PPG of a living body, but the invention is not limited thereto. The PPG sensor is typically a sensor device including a light emitting element that irradiates each of light rays (e.g., red light rays and infrared light rays) having two different wavelengths, and a light receiving element that receives the reflected light rays. The first sensor unit 30 may include other sensors instead of or in addition to the PPG sensor. For example, the first sensor unit 30 may be a sound sensor, a magnetic sensor, a voltage/current sensor, a heat flux sensor, a pressure sensor, or any combination thereof, which are adapted to measure heart sound, electrocardiogram (ECG), bioimpedance (BIA), galvanic skin reaction (GSR), or any combination thereof.

The first sensor unit 30 is connected to an external measurement device main body via, for example, a predetermined interface. For example, the first sensor unit 30 may be connected to an external measurement device main body via a cable (not shown), or may be wirelessly connected to the external measurement device main body by using a communication module (e.g., Wi-Fi (registered trademark) or Bluetooth (registered trademark) which is not shown) provided inside the base 10.

The adhering unit 40 is a member or a part having a predetermined adhesive force or a sticking force to be capable of adhering to the living body surface of the subject. In the present example, the adhering unit 40 itself forms the second sensor unit 50. The second sensor unit 50 may be, for example, an electrode sensor for measuring an electric signal of a living body such as an ECG. The electrode sensor is configured with, for example, a sticking pad in which an electrode element and a gel are integrated. The sticking force of the sticking pad causes the base 10 (i.e., the biological information measurement device 1) to adhere to and be held on the living body surface. Furthermore, in the present embodiment, as will be described later, for the sticking pad, a material and the surface area thereof are selected such that the sticking pad has a sticking force that does not detach the base 10 from the living body surface against the pressing force on the living body surface by the first sensor unit 30. The adhering unit 40 (sticking pad) can be configured to be attachable to and detachable from the base 10 by, for example, a snap button so that the adhering unit 40 (sticking pad) can be replaced in terms of hygiene or deterioration of the sticking force. The second sensor unit 50 may include other sensors instead of or in addition to the electrode sensor. For example, the second sensor unit 50 may include a sensor adapted to measure heart sound, bioimpedance (BIA), galvanic skin reaction (GSR), or a combination thereof.

The second sensor unit 50 may be connected to the external measurement device main body via, for example, a predetermined interface. For example, the second sensor unit 50 may be connected to an external measurement device main body via a cable (not shown), or may be wirelessly connected to the external measurement device main body by using a communication module provided inside the base 10.

In addition to the sticking pad as the second sensor unit 50, an auxiliary sticking pad for assisting the sticking force may be provided at a part of the bottom surface portion of the biological information measurement device 1. In order to make the adhering unit 40 have a sticking force, an adhesive may be applied to the adhering unit 40 at the time of use. In this example, the base 10 adheres with the sticking force of the adhering unit 40, but the present invention is not limited thereto, and for example, all or a part of the adhering unit 40 may be formed by a suction cup.

FIGS. 3 and 4 are views for illustrating a retractable pressing mechanism in the biological information measurement device according to the embodiment of the present invention, and more specifically, FIG. 3 shows a state where the first sensor unit 30 is at the retracted position, whereas FIG. 4 shows a state where the first sensor unit 30 is at the protruding position. These figures are partial cross sections in which a part of the base 10 is cut to illustrate how the first sensor unit 30 is moved by a retractable pressing mechanism. The retractable pressing mechanism of this example is configured such that the first sensor unit 30 is displaced from the retracted position to the protruding position and biased by a predetermined pressing force by a first pressing operation, and the predetermined pressing force is released and the first sensor unit 30 is displaced from the protruding position to the retracted position by a second pressing operation. The configuration of such a pressing mechanism is typically realized by including a known knocking mechanism, but is not limited thereto.

Specifically, as shown in FIG. 3, a pressing mechanism 60 of the biological information measurement device 1 includes, for example, a knocking unit 20, a cam main body 610 which is formed integrally with the base 10 and has a cam groove 612 and a tooth receiving unit 614, a rotary cam 620 which has cam teeth 622 that slide along the cam groove 612 along a forward movement of the knocking unit 20 and is rotatable around the cam main body 610, and a first elastic body 630 which elastically biases the knocking unit 20 and the rotary cam 620. The pressing mechanism 60 includes a stopper (not shown) for restricting the movement of the knocking unit 20 biased by the first elastic body 630 supported by a support unit 632. The pressing mechanism 60 further includes a holding unit 640 that come into contact with the rotary cam 620, and a second elastic body 650 which is provided between the holding unit 640 and the first sensor unit 30 and elastically biases the first sensor unit 30. The first elastic body 630 and/or the second elastic body 650 may be, for example, a compression spring. The second elastic body 650 is configured with, for example, four compression springs, and elastically supports the second sensor unit 50. Alternatively, the first elastic body 630 and/or the second elastic body 650 may be configured with an elastic body such as sponge, rubber, or an air piston.

The pressing mechanism 60 may include an adjusting unit (not shown) for adjusting the protrusion amount of the first sensor unit 30. The adjusting unit is configured to adjust the biasing force of the second elastic body 650 by, for example, a screw mechanism. Alternatively, the adjusting unit may be configured to adjust the biasing force of the second elastic body 650 by, for example, fitting the claw portion into any of the multi-stage notch portions.

In the pressing mechanism 60 configured as described above, in the initial state, the first sensor unit 30 is at a position (retracted position) slightly retracted from the bottom portion of the base 10. In this state, by the first pressing operation of the user with respect to the knocking unit 20, the knocking unit 20 moves forward (i.e., moves downward in the drawing) while resisting the biasing force of the first elastic body 630, and as a result by this, the rotary cam 620 also advances while the cam teeth 622 slide along the cam groove 612. At this time, the holding unit 640 in contact with the rotary cam 620 also advances, and accordingly, the first sensor unit 30 is advanced via the second elastic body 650. When the cam tooth 622 reaches the tip end portion of the cam groove 612, the rotary cam 620 is rotated by the cam tooth 622 disengaging from the cam groove 612 and engaging with the tooth receiving unit 614, and the knocking unit 20 is held in the advanced state. Therefore, the holding unit 640 coupled to the knocking unit 20 is also held in the advanced state, and accordingly, as shown in FIG. 4, the first sensor unit 30 is held at a position (protruding position) slightly protruding from the bottom portion of the base 10 while being elastically biased by the second elastic body 650.

In a state where the first sensor unit 30 is at the protruding position, by the second pressing operation of the user with respect to the knocking unit 20, the cam tooth 622 is disengaged from the tooth receiving unit 614, and the rotary cam 620 is rotated. By this, the cam tooth 622 is engaged with another cam groove 612, and the biasing force of the first elastic body 630 causes the rotary cam 620 to move backward while sliding the cam tooth 622 along the cam groove 612, and moves the knocking unit 20 backward. At this time, the holding unit 640 in contact with the rotary cam 620 also moves backward, and accordingly, the first sensor unit 30 moves backward via the second elastic body 650. Further, the cam tooth 622 comes into contact with the stopper, and then the backward movement of the knocking unit 20 is restricted, and the backward movement of the holding unit 640 is also restricted. Accordingly, the first sensor unit 30 is held at the original retracted position.

Therefore, in a case where the biological information measurement device 1 is attached to, for example, the living body surface of the subject, the biological information measurement device 1 adheres to the living body surface by a predetermined sticking force of the adhering unit 40 provided on the bottom surface portion of the base 10, and is reliably attached to the living body surface without being detached. In this state, when the holding unit 640 advances by the first pressing operation of the user with respect to the knocking unit 20, the first sensor unit 30 also advances while being elastically biased by the second elastic body 650. When coming into contact with the living body surface, the first sensor unit 30 is still biased by the second elastic body 650 and stops in a state of pressing the living body surface, while resisting the sticking force of the sticking pad. Therefore, the biological information measurement device 1 can measure a first biological signal when the first sensor unit 30 comes into contact with the living body surface with an appropriate pressing force, and the biological information measurement device 1 can measure a second biological signal at the same time since the second sensor unit 50 adheres by the sticking pad.

In a case where the biological information measurement device 1 is removed from the living body surface of the subject, for example, by the second pressing operation of the user with respect to the knocking unit 20, the compressive force of the first elastic body 630 is released, and the first sensor unit 30 moves backward to the retracted position. The user can further remove the biological information measurement device 1 by peeling off the biological information measurement device 1 adhering to the living body surface by the sticking pad.

As described above, according to the present embodiment, while the biological information measurement device 1 adheres to the living body surface of the subject by a predetermined sticking force by the sticking pad on the bottom surface portion, the first sensor unit 30 can be easily attached by an appropriate pressing force, the biological information measurement device 1 does not fall off accidentally, and the biological signal can be reliably measured. Among others, for example, in a case where the first sensor unit 30 is a PPG sensor or the like, unintended scattering of reflected light can be prevented by an appropriate pressing force on the living body surface, and/or the biological signal can be more reliably measured as the biological signal is emphasized with an appropriate compression of arterial blood.

According to the present embodiment, since the biological information measurement device 1 is designed on the assumption that the biological information measurement device 1 adheres to the living body surface of the subject, a contact area with the living body surface can be sufficiently ensured. Therefore, the first sensor unit 30 having a relatively large size can be adopted, and further, the first sensor unit 30 can also be used together with the second sensor unit 50.

### [Second Embodiment]

In the present embodiment, various modification examples of the biological information measurement device including the pressing mechanism 60 of another example instead of the knocking mechanism as described above will be described.

### (Modification Example 1)

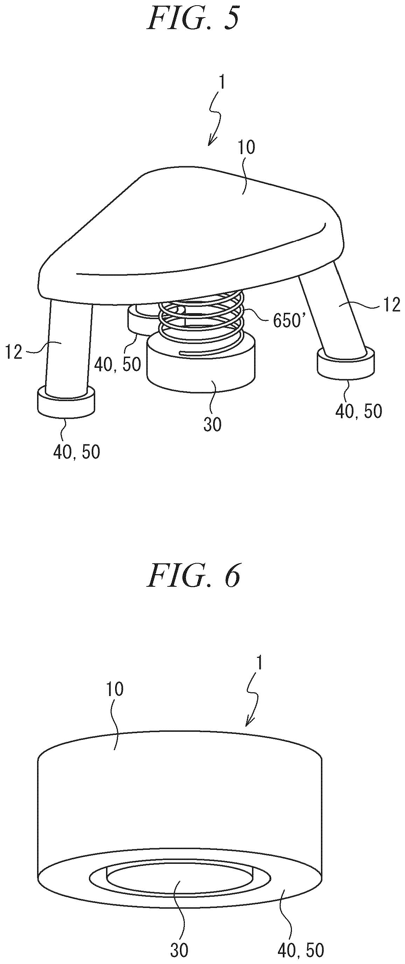

FIG. 5 is a view showing an example of a configuration of the biological information measurement device according to an embodiment of the present invention. Specifically, as shown in this figure, the biological information measurement device 1 of the present embodiment includes, for example, the base 10, the first sensor unit 30, the adhering unit 40 including the second sensor unit 50, and the pressing mechanism 60. The pressing mechanism 60 of the present example is configured to include an elastic body 650' that elastically biases the first sensor unit 30, and three stem units 12 that support the base 10 at each of one end portions of the stem units 12, and the adhering units 40 are provided at the other end portions of the stem units 12.

In this example, the base 10 is formed in a flat and substantially triangular columnar shape, and the three stem units 12 are arranged at corner parts, but the present invention is not limited thereto. For example, the external shape of the base 10 may be a flat disk shape or an elliptical disk shape. The first sensor unit 30 is provided to extend from the substantially central portion of the base 10 to be surrounded by the three stem units 12. As an example, the first sensor unit 30 is provided such that the geometric gravity center position of the second sensor unit 50 substantially matches the first sensor unit 30 within the virtual plane with respect to the living body surface.

In such a configuration, when the adhering unit 40 comes into contact with the living body surface of the subject, while the adhering unit 40 adheres with the sticking force to hold the biological information measurement device 1, the first sensor unit 30 is elastically biased by the elastic body 650' against the sticking force and presses the living body surface. Therefore, in this state, similarly to the above-described embodiment, the first sensor unit 30 can measure the first biological signal while being in contact with the living body surface with an appropriate pressing force, and the second sensor unit 50 can measure the second biological signal at the same time since the second sensor unit 50 adheres by the adhering unit 40.

In the present disclosure, the elastic body 650' is described as a coil spring member, but the present invention is not limited thereto, and the elastic body 650' may be, for example, a plate spring member, an air spring member, or a flexible member. Similarly, the stem unit 12 is described as a rigid member, but the present invention is not limited thereto, and the stem unit 12 may be, for example, a flexible member. Alternatively, instead of the elastic body 650', the base 10 may be supported by a plurality of stems or support legs.

### (Modification Example 2)

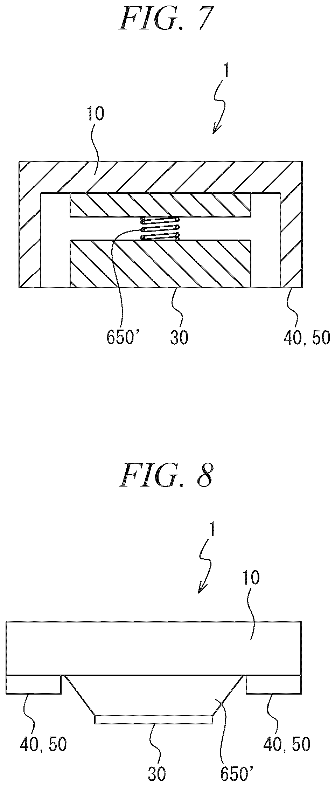

FIGS. 6 and 7 are views showing an example of a configuration of the biological information measurement device according to the embodiment of the present invention, and specifically, FIG. 6 is an external downward perspective view, and FIG. 7 is a side sectional view. As shown in these figures, the biological information measurement device 1 of the present embodiment includes, for example, the substantially columnar or substantially disk-shaped base 10, the adhering unit 40, and the pressing mechanism 60. The adhering unit 40 is provided along the annular bottom surface portion of the base 10. In a state where the biological information measurement device 1 is not in contact with the living body surface of the subject, the first sensor unit 30 is configured such that a contact surface thereof slightly protrudes from a contact surface of the surrounding adhering unit 40 (second sensor unit 50). Similarly to the above-described embodiment, when the adhering unit 40 comes into contact with the living body surface of the subject, while the adhering unit 40 adheres with the sticking force to hold the biological information measurement device 1, the first sensor unit 30 is elastically biased by the elastic body 650' against the sticking force and presses the living body surface.

### (Modification Example 3)

FIG. 8 is a view illustrating an example of the configuration of the biological information measurement device according to the embodiment of the present invention. Specifically, in the biological information measurement device 1 shown in this figure, the pressing mechanism 60 is configured with the elastic body 650' made of a flexible member such as rubber or sponge. With such a configuration, when the top end portion (not shown) of the base 10 is pressed, the first sensor unit 30 is compressed while pressing the living body surface, and the adhering unit 40 comes into contact with and adheres to the living body surface of the subject.

### (Modification Example 4)

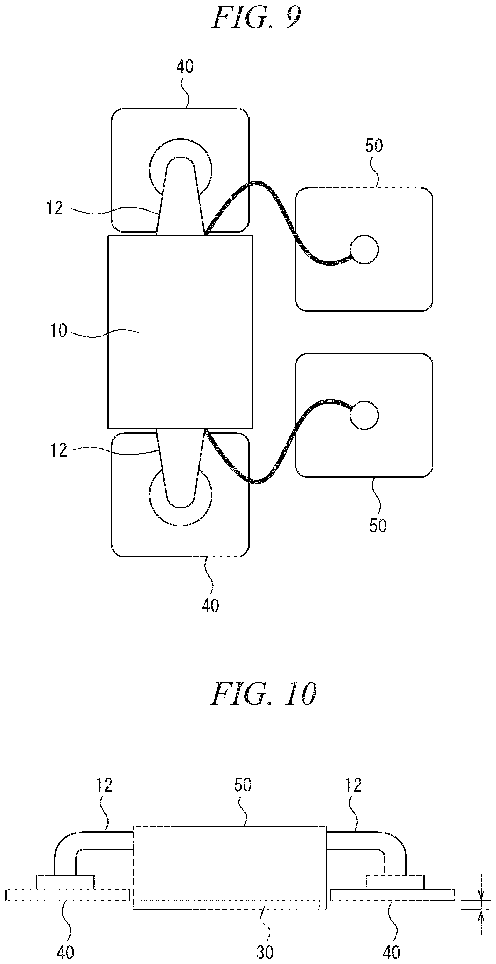

FIG. 9 is a plan view showing an example of a configuration of the biological information measurement device according to the embodiment of the present invention, and FIG. 10 is a side view of the base part of the biological information measurement device illustrated in FIG. 9.

Specifically, as shown in this figure, in the biological information measurement device 1 of the present example, while the first sensor unit 30 is provided at the bottom surface portion of the base 10, the second sensor unit 50 is provided separately from the base 10. In other words, in this example, the second sensor unit 50 is configured independently of the adhering unit 40. The second sensor unit 50 may be connected to the base 10 by, for example, a cable.

The base 10 includes the stem unit 12 which is integrally formed, extends in a substantially horizontal direction from the side end portion of the main body of the base 10, and is further bent downward, and accordingly, a part of the stem unit 12, which extends in a substantially horizontal direction, is configured to be elastically deflected. In other words, in this example, the stem unit 12 acts as the elastic body 650'. At the tip end portion of the stem unit 12, the adhering unit 40 for making the biological information measurement device 1 adhere to the living body surface is provided. As is apparent from FIG. 10, the surface of the base 10 including the bottom surface portion slightly protrudes from the surface including the adhering unit 40. Therefore, in a case where the biological information measurement device 1 adheres to the living body surface by the adhering unit 40, the bottom surface portion of the base 10 provided with the first sensor unit 30 is pressed to the living body surface by a force that depends on the deflection amount of the stem unit 12.

Therefore, even in the above-described example, when the adhering unit 40 comes into contact with the living body surface of the subject, while the adhering unit 40 adheres with the sticking force to hold the biological information measurement device 1, the first sensor unit 30 presses the living body surface with the deflecting force of the stem unit 12 against the sticking force. Therefore, in this state, similarly to the above-described embodiment, the first sensor unit 30 can measure the first biological signal while coming into contact with the living body surface with an appropriate pressing force, and the second sensor unit 50 formed of a separate body from the base 10 can also measure the second biological signal at the same time.

### (Modification Example 5)

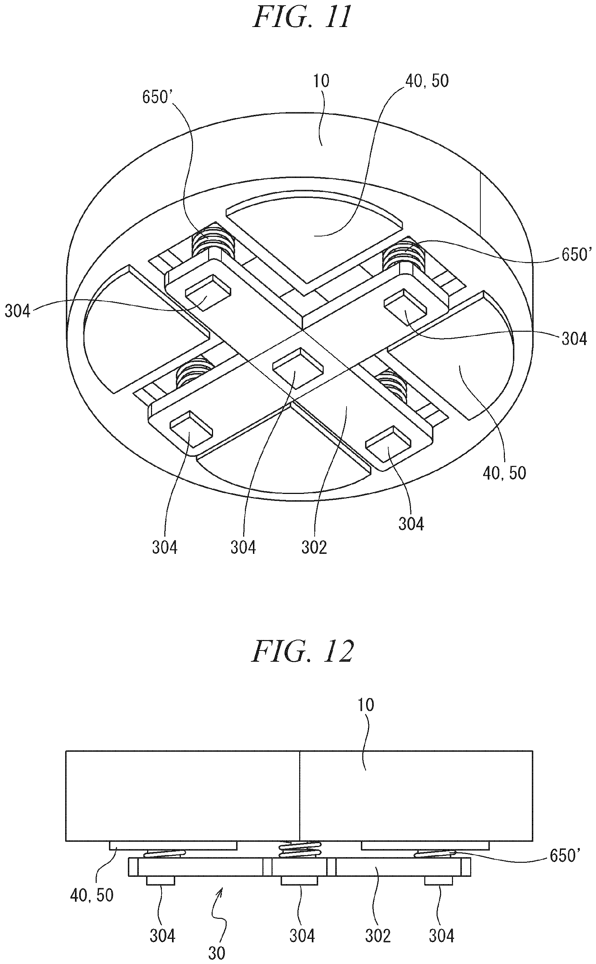

FIG. 11 is a downward perspective view showing an example of an external configuration of the biological information measurement device according to an embodiment of the present invention, and FIG. 12 is a side view showing an example of the external configuration of the biological information measurement device shown in FIG. 11.

As shown in these figures, the biological information measurement device 1 of the present example is configured to include the substantially disk-shaped base 10, but the invention is not limited thereto. The base 10 is formed in a size having, for example, a diameter of approximately 40 mm and a height of approximately 10 mm, but the invention is not limited thereto. On the bottom surface portion of the base 10, for example, the first sensor unit 30 configured to include a substantially cross-shaped board 302 is elastically provided via the elastic body 650'. In the bottom surface portion of the base 10, the adhering unit 40 is provided at a part (i.e., a quadrant-shaped part or a fan-shaped part) other than the first sensor unit 30.

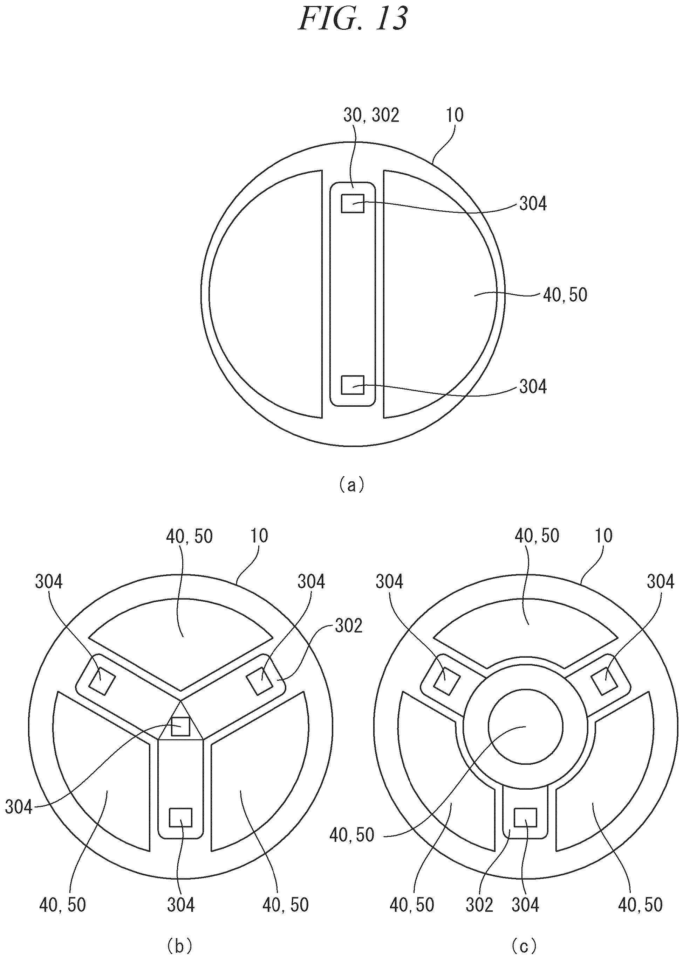

The first sensor unit 30 includes a plurality of (five in this example) sensors 304 provided on the board 302. In the present disclosure, the sensor 304 may be an individual sensor configuration element that configures an active sensor, such as a light emitting element and a light receiving element. The board 302 may be, for example, a rigid board in whole or a part thereof, or may be a flexible board, and basically, may support and appropriately press the sensor 304. In the present example, the first sensor unit 30 is formed in a substantially cross shape, but the present invention is not limited thereto, and the shape may have an elongated plate shape or a substantially Y shape (see FIG. 13). The sensor 304 is disposed, for example, at each end portion and a central portion of the first surface (i.e., the surface facing the living body surface of the subject) of the board 302. Some or all of the sensors 304 are, for example, PPG sensors including a light emitting element that irradiates light having two different wavelengths and a light receiving element that receives the reflected light. In this example, the sensors 304 are arranged geometrically symmetrically on the board 302, but may be disposed asymmetrically.

The elastic body 650' is configured with, for example, a plurality of (five in this example) coil springs such that the board 302 can be elastically supported stably, but the elastic body 650' is not limited thereto as described above. In this example, the elastic body 650' elastically supports each end portion and the central portion of the second surface opposite to the first surface of the board 302 at the position corresponding to the PPG sensor, and thus, each sensor 304 can be pressed with an appropriate pressing force without impairing the characteristics of following the shape of the living body surface.

Similarly to the above-described embodiment, the adhering unit 40 may be configured to include the second sensor unit 50. In this example, the adhering unit 40 is formed in the region of four quarter circles, but the present invention is not limited thereto, and the adhering unit 40 may also be formed at the peripheral edge part. Typically, the shape and size of the adhering unit 40 depend on the relationship between the shape of the base 10 and the shape of the first sensor unit 30.

For example, as shown in (a) of FIG. 13, in a case where the first sensor unit 30 having an elongated plate shape is provided on the bottom surface of the substantially disk-shaped base 10, the adhering unit 40 having a substantially semicircular shape in a plan view can be selected. As shown in (b) of FIG. 13, in a case where the first sensor unit 30 having a substantially Y shape is provided on the bottom surface of the substantially disk-shaped base 10, the adhering unit 40 having a substantially fan shape of which a center angle is approximately 60 degrees in a plan view can be selected. As shown in (c) of FIG. 13, in addition to the substantially fan-shaped adhering unit 40, the biological information measurement device 1 may also have the adhering unit 40 provided at the central portion of the substantially Y-shaped first sensor unit 30.

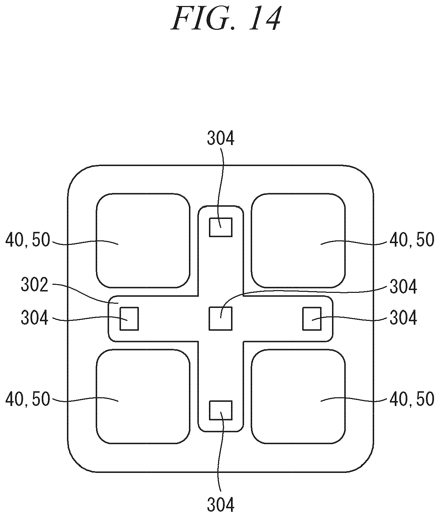

Alternatively, for example, as shown in FIG. 14, in a case where the first sensor unit 30 having a substantially cross shape is provided on the bottom surface of the flat and substantially rectangular plate-shaped base 10, the adhering unit 40 having a substantially rectangular shape in a plan view may be selected. Although not shown, the substantially cross-shaped first sensor unit 30 may be provided to be positioned on the diagonal line of the substantially rectangular plate-shaped base 10, and the adhering unit 40 may be provided at the remaining part.

As described above, when the adhering unit 40 comes into contact with the living body surface of the subject, while the adhering unit 40 adheres with the sticking force to hold the biological information measurement device 1, the first sensor unit 30 reliably presses the living body surface with the pressing force of the elastic body 650' against the sticking force. Therefore, in this state, similarly to the above-described embodiment, the first biological signal can be measured while the first sensor unit 30 is in contact with the living body surface with an appropriate pressing force. Even if the base 10 has a substantially rectangular plate shape, there is no protruding part, and therefore, there is no inconvenience that the biological information measurement device 1 is detached by being caught by clothes or the like while being attached to the subject.

### (Modification Example 6)

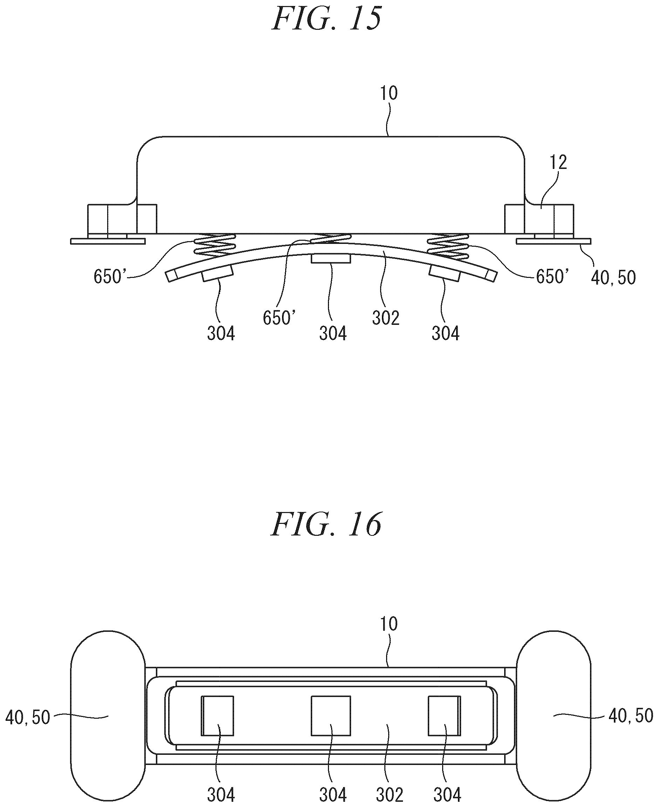

FIG. 15 is a side view showing an example of an external configuration of the biological information measurement device according to an embodiment of the present invention, and FIG. 16 is a bottom view showing an example of the external configuration of the biological information measurement device shown in FIG. 15.

As shown in these figures, the biological information measurement device 1 of this example is configured to include the flat and substantially rectangular parallelepiped-shaped base 10, and the stem units 12 extending from both end portions of the base 10. The base 10 is formed in a size having, for example, a length of approximately 40 mm in the longitudinal axis direction, a length of approximately 10 mm in the lateral axis direction, and a height of approximately 10 mm, but the invention is not limited thereto. On the bottom surface portion of the base 10, the first sensor unit 30 is provided to be elastically supported by the plurality of (three in this example) elastic bodies 650' substantially along the outer shape of the base 10.

Similarly to the above-described example, the first sensor unit 30 is configured to include the board 302. The board 302 is formed in a size having, for example, a length of approximately 30 mm in the longitudinal axis direction, a length (width) of approximately 10 mm in the lateral axis direction, and a height of approximately 10 mm. The plurality of sensors 304 is provided on the board 302. In the present example, the plurality of sensors 304 is arranged at both end portions and the central portion of the board 302, but the present invention is not limited thereto. For example, two sensors 304 may be disposed at one end portion of the board 302, and one sensor 304 may be disposed at the other end portion.

The elastic body 650' is configured with, for example, a plurality of (three in this example) coil springs such that the board 302 can be elastically supported stably, but the invention is not limited thereto as described above. In this example, the board 302 has a curved portion formed to be curved or arched without contacting with the living body surface of the subject. When the curved portion of the board 302 comes into contact with the living body surface of the subject, the curved portion can deflect following the shape of the living body surface and press the sensor 304 by applying the pressing force of the elastic body 650'. The board 302 only needs to be a member that deflects following the shape of the living body surface, and the degree of rigidity or flexibility thereof can be appropriately selected.

The adhering unit 40 is supported by the stem units 12 extending from both end portions of the base 10. The stem unit 12 is formed in a size having, for example, a length of approximately 5 mm in the extending direction and a height of approximately 3 mm, and the adhering unit 40 has, for example, an oval shape having a maximum width of approximately 20 mm. In this example, a configuration is described in which the stem unit 12 is provided on the flat and substantially rectangular parallelepiped-shaped base 10, but the invention is not limited thereto, and the stem unit 12 may be provided on the base 10 having various shapes as described above.

As described above, in this example, when the adhering unit 40 comes into contact with the living body surface of the subject, while the adhering unit 40 adheres with the sticking force to hold the biological information measurement device 1, the first sensor unit 30 reliably presses the living body surface with the deflecting force of the board 302 and the pressing force of the elastic body 650' against the sticking force. Therefore, in this state, similarly to the above-described embodiment, the first biological signal can be measured while the first sensor unit 30 is in contact with the living body surface with an appropriate pressing force. Since the base 10 has a thin shape and has no protruding part, there is no inconvenience that the biological information measurement device 1 is detached by being caught by clothes or the like while being attached to the subject.

### [Third Embodiment]

In the present embodiment, an example of a configuration is described in which the geometrical positional relationship between the first sensor unit and the adhering unit is defined in the biological information measurement device 1 according to the above-described embodiment.

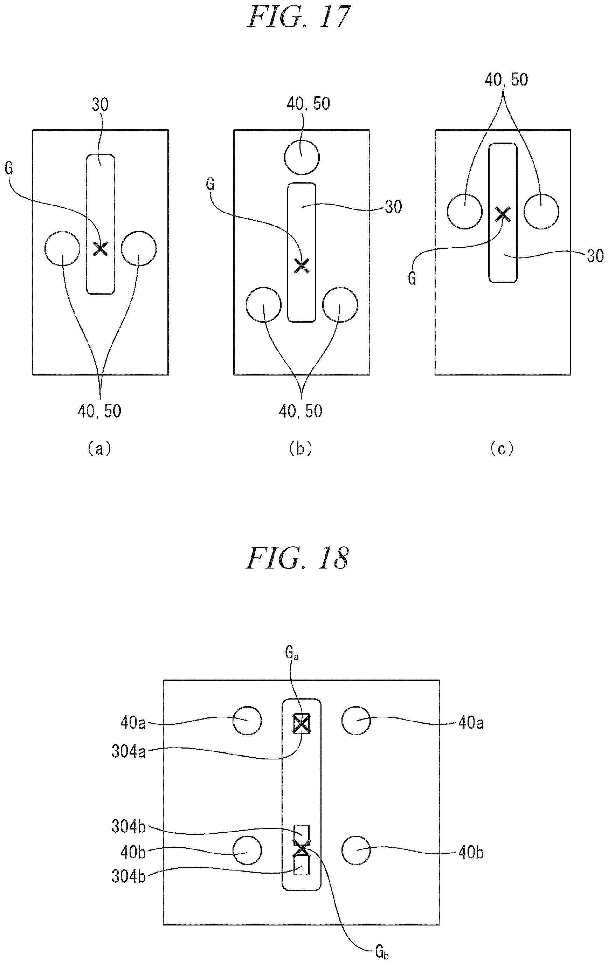

FIG. 17 shows views for illustrating examples of the geometrical positional relationship between the first sensor unit and the adhering unit in the biological information measurement device according to an embodiment of the present invention. Specifically, as shown in this figure, the first sensor unit 30 and the adhering unit 40 are provided on the base 10 such that the gravity center of the sticking surface of the adhering unit 40 is positioned within the contact surface of the first sensor unit 30 (the board 302) on the virtual plane including the living body surface of the subject.

More specifically, in (a) of FIG. 17, an example is shown in which the two adhering units 40 are provided in the vicinity of the long side end portions of the first sensor unit 30, respectively. Therefore, the gravity centers of the sticking surfaces of the two adhering units 40 are positioned within the contact surface (more accurately, on the longitudinal axis) of the board 302 of the first sensor unit 30.

In (b) of FIG. 17, an example is shown in which three adhering units 40 are provided around the first sensor unit 30. The gravity center position of the sticking surface of the adhering unit 40 matches the gravity center position of the triangle with each of the sticking surfaces as the apex, and is positioned within the contact surface of the first sensor unit 30.

In (c) of FIG. 17, an example is shown in which the first sensor unit 30 and the adhering unit 40 are provided on the base 10 such that the gravity center position of the contact surface of the first sensor unit 30 and the gravity center position of the adhering surface of the adhering unit 40 substantially match each other.

Alternatively, although not shown, the biological information measurement device 1 may be configured such that the gravity center position of the contact surface of the first sensor unit 30 and the gravity center position of the plurality of elastic bodies 650' substantially match each other.

FIG. 18 is a view for illustrating an example of the geometrical positional relationship between the first sensor unit and the adhering unit in the biological information measurement device according to the embodiment of the present invention. More specifically, this figure illustrates the positional relationship between a gravity center G of the sensors 304 (304a, 304b) provided in the first sensor unit 30 and the gravity center of the sticking surface of the adhering unit 40.

As shown in this figure, the gravity center position of the sticking surface of the plurality of adhering units 40 associated with one or a plurality of sensors 304 close to each other substantially matches the gravity center position of the sensor 304. Although not shown, for example, the adhering unit 40 is supported by the stem portion 12 extending from the base 10. It is noted that "the sticking surface of the plurality of adhering units 40 associated with one or a plurality of sensors 304 close to each other" means the sticking surface of the adhering unit 40 positioned in the vicinity of one or a plurality of sensors 304 near each other, and the adhering units 40 in which the sticking force is closely related to the pressing force on the sensor 304, among the plurality of adhering units 40. In this example shown in the figure, sticking surfaces 40a of the two adhering units 40 are associated with the sensor 304a, and sticking surfaces 40b of the other two adhering units 40 are associated with the two sensors 304b. The gravity center positions of the two sticking surfaces 40a substantially match the gravity center position of one sensor 304a, and the gravity center positions of the two sticking surfaces 40b substantially match the gravity center positions of the two sensors 304b.

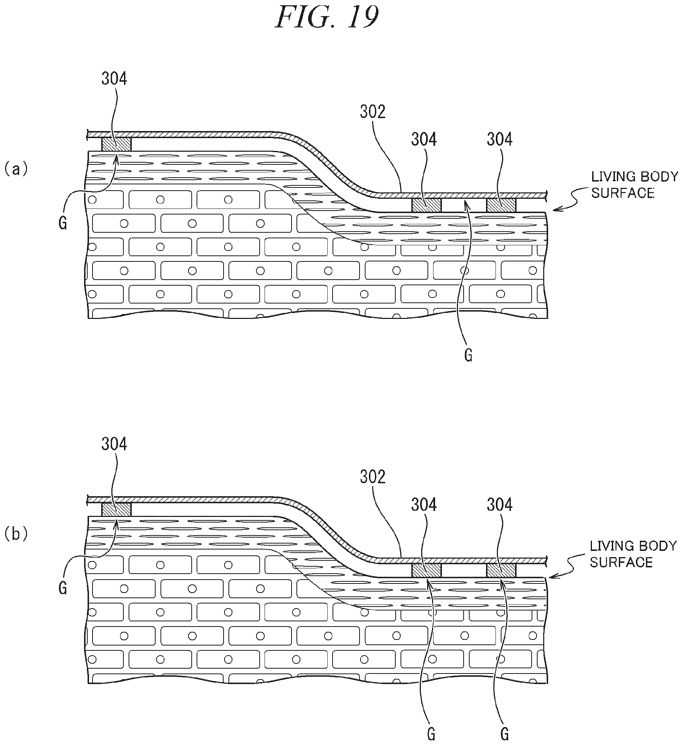

The board 302 that configures the first sensor unit 30 may be a flexible board as described above. Therefore, in a case where the gravity center position of the sticking surface of the adhering unit 40 substantially matches the sensor 304, as shown in FIG. 19, the sensor 304 reliably presses the living body surface of the subject while the shape of the board 302 deflects following to the step or curved surface shape of the living body surface of the subject.

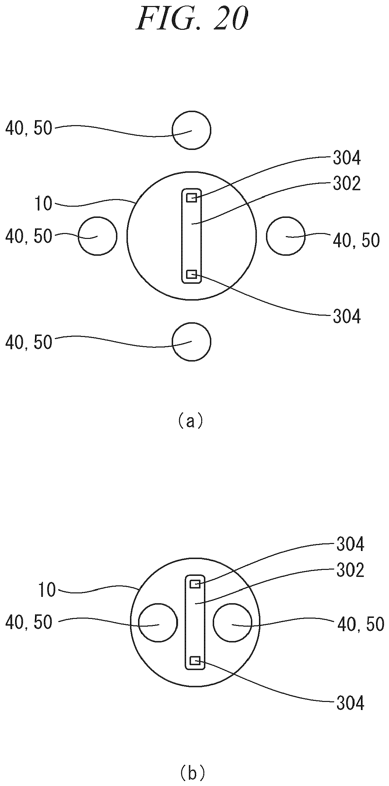

FIG. 20 shows views for illustrating an example of the geometrical positional relationship between the first sensor unit and the adhering unit in the biological information measurement device according to the embodiment of the present invention. Specifically, the figure shows the distance relationship from the gravity center position of the first sensor unit 30 to the sticking surface of the adhering unit 40 on the virtual plane including the living body surface of the subject. In the present example, the gravity center position of the sticking surface is conditioned to be included in the contact surface of the first sensor unit.

In this example, the maximum distance from the gravity center position of the first sensor unit 30 to the sticking surface of each adhering unit 40 is approximately 10 cm. In other words, the pair of adhering units 40 centered on the first sensor unit 30 are disposed to be separated by a maximum of approximately 20 cm. Accordingly, deterioration of the SN ratio of the biological signal (for example, ECG signal) detected by the second sensor unit 50 provided in the adhering unit 40 can be suppressed.

The geometrical positional relationship between the first sensor unit 30 and the adhering unit 40 is not limited to the above-described example, and various cases are assumed. For example, in a case where the first sensor unit 30 is configured with the plurality of boards 302, the first sensor unit 30 and the adhering unit 40 may be disposed such that the gravity center position of the sticking surface of the adhering unit 40 substantially matches the gravity center position of the contact surface of the first sensor unit 30.

As described above, according to the present embodiment, when the adhering unit 40 comes into contact with the living body surface of the subject, while the adhering unit 40 adheres with the sticking force to hold the biological information measurement device 1, the first sensor unit 30 reliably presses the living body surface with the pressing force of the elastic body 650' against the sticking force. Therefore, in this state, similarly to the above-described embodiment, the first biological signal can be measured while the first sensor unit 30 is in contact with the living body surface with an appropriate pressing force.

Each of the above-described embodiments is an example for describing the present invention, and is not intended to limit the present invention only to these embodiments. The present invention can be implemented in various forms without departing from the spirit of the present invention.

For example, in the methods disclosed herein, steps, actions, or functions may be performed in parallel or in a different order, as long as the results are not inconsistent. The steps, actions, and functions described are provided merely as examples, and some of the steps, actions, and functions can be omitted or coupled to each other without departing from the gist of the invention, or other steps, actions, or functions may be added.

Although various embodiments are disclosed in the present specification, specific features (technical matters) in one embodiment can be added to other embodiments while being appropriately improved, or can be replaced with specific features in other embodiments, and these aspects can also be included in the gist of the invention.

### Industrial Applicability

The present invention can be widely used in the field of devices for measuring biological information. For example, the present invention can be used in various devices for measuring PPG, heart sound, blood pressure, heart rate, galvanic skin reaction, and the like.

### Reference Signs List

- 1: biological information measurement device
- 10: base
- 12: stem unit
- 20: knocking unit
- 30: first sensor unit
- 302: board
- 304: sensor, sensor configuration element
- 40: adhering unit
- 50: second sensor unit
- 60: pressing mechanism
- 610: cam main body
- 612: cam groove
- 614: tooth receiving unit
- 620: rotary cam
- 622: cam teeth
- 630: first elastic body
- 640: holding unit
- 650: second elastic body
- 650': elastic body

## Claims

1. A biological information measurement device comprising:
a base;
a first sensor unit provided in a displaceable manner with respect to the base, and including a plurality of sensors that measure biological information of a subject;
a pressing unit that elastically presses the first sensor unit to a living body surface of the subject; and
a plurality of adhering units, provided on the base, that adheres to the living body surface, wherein
the pressing unit is configured to press the plurality of sensors of the first sensor unit against the base adhering to the living body surface by an adhesive force of the plurality of adhering units.

2. The biological information measurement device according to claim 1, wherein
the pressing unit further includes an adjusting unit that adjusts an amount of protrusion of the first sensor unit with respect to a bottom surface of the base.

3. The biological information measurement device according to claim 1 or 2, wherein
at least some of the plurality of sensors in the first sensor unit are PPG sensors that optically measure PPG.

4. The biological information measurement device according to any one of claims 1 to 3, wherein
the plurality of adhering units includes a second sensor unit.

5. The biological information measurement device according to claim 4, wherein
the second sensor unit is formed of a separate body from the plurality of adhering units.

6. The biological information measurement device according to claim 4 or 5, wherein
the second sensor unit includes an electrode pad that measures a bioelectric signal.

7. The biological information measurement device according to any one of claims 1 to 6, wherein
the pressing unit includes
a knocking unit that displaces depending on a pressing operation, and
a first elastic body that elastically biases the knocking unit,
the first sensor unit is provided to move in conjunction with movement of the knocking unit, and
in response to a first pressing operation with respect to the knocking unit, the knocking unit advances and the first sensor unit at a retracted position advances and is positioned to protrude from a bottom surface portion of the base, and in response to a second pressing operation with respect to the knocking unit, the knocking unit retracts and the first sensor unit protruding from the bottom surface portion of the base moves to the retracted position.

8. The biological information measurement device according to claim 7, wherein
the pressing unit further includes a second elastic body that elastically biases the first sensor unit.

9. The biological information measurement device according to any one of claims 1 to 6, wherein
the pressing unit includes a plurality of elastic bodies that elastically biases the first sensor unit, and
each of the plurality of elastic bodies is provided on a second surface opposite to a first surface of the first sensor unit on which the plurality of sensors is provided.

10. The biological information measurement device according to any one of claims 1 to 9, further comprising:
a plurality of support units, provided on the base, that support the plurality of adhering units, respectively.

11. The biological information measurement device according to any one of claims 1 to 10, wherein
a gravity center position with respect to the plurality of adhering units is included in the first sensor unit in a virtual plane including the living body surface.

12. The biological information measurement device according to claim 11, wherein
the gravity center position with respect to the plurality of adhering units substantially matches a gravity center position with respect to the plurality of sensors.

13. The biological information measurement device according to claim 9, wherein
a gravity center position of the first sensor unit substantially matches a gravity center position of the plurality of elastic bodies.

14. The biological information measurement device according to any one of claims 1 to 13, wherein
the first sensor unit includes a board on which the plurality of sensors is mounted, and the board is a flexible board.

15. The biological information measurement device according to any one of claims 1 to 13, wherein
the first sensor unit includes a board on which the plurality of sensors is mounted, the board is formed in a curved portion, and the curved portion is configured to come into contact with the living body surface following the shape of the living body surface.
